# EUROPEAN PATENT APPLICATION

(11) **EP 4 299 000 A1**
(43) Date of publication of application: **03.01.2024**
(21) Application number: 22020308.7
(22) Date of filing: 30.06.2022
(51) Int. Cl.: A61B 5/021, A61B 5/0265, A61B 5/05

(54) **PORTABLE ULTRASOUND BASED ARTERIAL CIRCULATION EVALUATION METHOD**

(71) Applicant: CERAGOS Electronics & Nature, 06800 Cagnes-sur-Mer (FR)
(72) Inventor: CERASANI, Luca, 06800 Cagnes-sur-Mer (FR)

(57) **Abstract**

Continuous noninvasive arterial blood pressure measurement (CNAP), also called Vascular Unloading Technic, is the current portable, continuous and non-invasive methodology to measure blood pressure. It is based on measurement of finger blood volume changes using infrared light. This methodology is commonly used in Hospitals even if it can be inaccurate in some cases and requires a pressure servo loop.

A new methodology/process is presented to continuously, directly and non-invasively measure blood pressure based on the combination of a magnetizing coil, a magnetic sensor and Ultrasound emitter. Previously magnetized blood flow is monitored by a magnetic sensor such as a search coil or a magneto-resistor. An Ultrasound beam opposing the blood flow is temporarily applied modifying blood vessel pressure and then changing the monitored blood flow. The blood pressure is determined by evaluation of the variation of the blood flow velocity in relation with the (approximated) change of blood pressure.

This new sensing methodology has a low impact on blood vessel circulation and eliminates the need of the pressure servo loop. It can be applied to several arteries such as the Brachial and Radial arteries. It can also be extended to allow sensing of echo Ultrasounds for determination of blood flow velocity and artery diameter using Doppler effect and then providing a consistent picture of blood circulation in the artery i.e. Pressure, Blood Flow, artery diameter and macroscopic evaluation of Blood Viscosity.

## Description

### Prior art:

[D01] Several technologies are or have been used to measure the blood flow such as Thermodilution, Electromagnetic Flow Probe, Doppler Catheter, Doppler Echocardiography, Laser Doppler Flowmetry, Impedance Cardiography, Tomography, Pulse Oximetry, Magnetic Resonance Imaging (MRI) but none of them offer a portable solution. In addition there are few technologies for continuous measurement of the blood flow velocity.

[D02] Continuous noninvasive arterial blood pressure measurement (CNAP) also called Vascular Unloading Technic is based on measurement of finger blood volume changes by infrared light sensors. The main drawbacks of this method are:
- Blood flow is evaluated by infrared light sensor
- Limited to finger artery (cannot be easily transposed to other arteries)
- Requires a pressure servo loop

[D03] Modulated Magnetic Signature of Blood (MMSB) uses an applied magnetic field to magnetize blood cells and then measure the magnetic field generated by the blood flow using a magnetic sensor. Even if MMSB has been proposed to be used for blood pressure measurement too, it suffers of the lack of linearity and reproducibility [4] [2].

### [D04] References:

[1] Phua, C. T., Lissorgues, G., and Mercier, B., 2009, "Non-Invasive Acquisition of Blood Pulse using Magnetic Disturbance Technique," International Conference of Biomedical Engineering (ICBME), pp. 786-789
[2] Francy L. Sinatra University of South Florida "Understanding the Interaction Between Blood Flow and an Applied Magnetic Field" (2010).

### Description of the drawings:

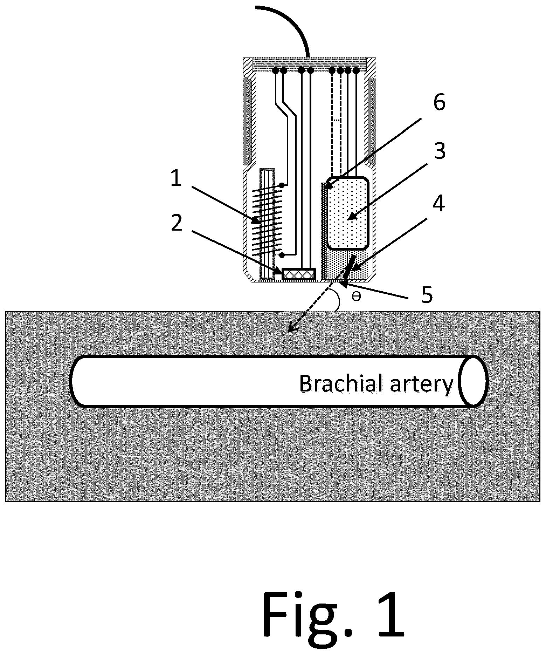

[D05] Fig. 1 is a diagram describing the structure of an embodiment based on the Portable UltraSound based Arterial Circulation evaluation process (PUSAC for short) primarily used for measurement of arterial pressure, comprising:
- a coil (1) positioned above the artery and generating a magnetic field perpendicular to the axis of the artery,
- a magnetic sensor (2) monitoring the variation of the magnetic field generated by the magnetized blood flow,
- an Ultrasound emitter (3) temporarily delivering a beam that is deflected by a mirror (4) to oppose it to the blood flow.

### Description of the embodiments:

[D06] The Portable UltraSound based Arterial Circulation evaluation process (PUSAC for short) can be basically implemented using five elements (also see joined figure 1):
- A Coil to slightly and continuously magnetize the blood,
- A magnetic sensor such as a Giant Magneto-Resistor (GMR) or a search Coil is used to capture the variation of the blood flow in a similar way than on MMSB,
- An Ultrasound Emitter generating variable intensity and low frequency Ultrasounds (from 1 to 3 MHz or lower) creating a variation of the blood pressure in the artery and then modifying the blood flow velocity which change is captured by the magnetic sensor.
- An Ultrasound mirror could be used to have the ultrasound beam almost aligned with the artery and opposing the blood flow,
- An Electronic circuit is used to control and synchronize all elements, and provide a visual output of the measured blood pressure.

[D07] The blood pressure is determined by measuring the variation of the blood flow velocity induced by the opposing Ultrasound beam and applying the same percentage of variation to the blood pressure, knowing an approximative value of the Ultrasound beam pressure (and assuming a low percentage of variation).

[D08] The blood pressure measure can be repeated several times during the whole cardiac cycle providing an almost continuous measurement of the blood pressure.

[D09] The proposed process can be extended to allow sensing of echo Ultrasounds for determination of both the artery diameter and the blood flow velocity and then providing a consistent picture of blood circulation in the artery i.e. Blood Pressure, Blood Flow velocity, artery diameter and then macroscopic evaluation of Blood Viscosity.

[D10] Ultrasound waves influence can be replaced by a manually or automatically operated Force Sensor positioned above the artery.

## Claims

1. A new method/process to continuously, directly and non-invasively measure blood pressure based on an Ultrasound emitter combined with magnetic detection of blood flow. It includes the following steps:
• Analyzed artery is magnetized by a strong, but harmless Magnetic Field that is provided by a coil.
• Blood flow velocity is permanently and roughly evaluated according to the induced variation of the Magnetic Field detected by a magnetic sensor such as a Search Coil or an Anisotropic Magneto-Resistance (AMR).
• An Ultrasound emitter will briefly generate Ultrasound Waves opposing the blood flow which is monitored in parallel.
• The blood flow pressure can be determined knowing the (small) decrease of the blood flow pressure due to the Ultrasound beam, and relating it to the proportionally identical measured variation of the blood flow velocity.

2. The blood pressure measure can be repeated several times during the whole cardiac cycle to get a temporal graph.

3. Alternative methodologies for more accurate measurement of blood flow using other magnetic or electromagnetic sensing approaches such as double magnetizing coil or variable magnetization are possible.

4. The proposed system can be extended to allow sensing of echo Ultrasounds for determination of both artery diameter and blood flow velocity, and then providing a consistent picture of blood circulation in the artery i.e. Blood Pressure, Blood Flow velocity, artery diameter and then macroscopic evaluation of Blood Viscosity.

5. Ultrasound waves influence can be replaced by a manually or automatically operated Force Sensor positioned above the artery.
